# EUROPEAN PATENT APPLICATION

(11) **EP 3 351 955 A1**
(43) Date of publication of application: **25.07.2018**
(21) Application number: 17020020.8
(22) Date of filing: 20.01.2017
(51) Int. Cl.: G01R 33/48, A61B 6/03, A61B 6/00, G01T 1/16, G01R 33/34, G01R 33/422, G01T 1/29

(54) **RF-SHIELDED HYBRID MR SYSTEM**

(71) Applicant: Klomp, Dennis, 5301 KR Zaltbommel (NL); Steensma, Bart R., 5301 KR Zaltbommel (NL); Versteeg, Edwin, 5301 KR Zaltbommel (NL); Rivera, Debra Strick, 5301 KR Zaltbommel (NL); MR Coils, BV, 5301 KR Zaltbommel (NL)
(72) Inventor: KLOMP, Dennis, 5301 KR Zaltbommel (NL); STEENSMA, Bart R., 5301 KR Zaltbommel (NL); VERSTEEG, Edwin, 5301 KR Zaltbommel (NL); RIVERA, Debra Strick, 5301 KR Zaltbommel (NL)

(57) **Abstract**

The herein described invention relates to a method for incorporating a sensor and/or actuator system within an MRI-bore of an MRI-system. RF transmit systems comprise of coils and/or antennas composed of conductive structures contained within an RF shield. Sensors and or actuator systems can be placed between the conductors of the RF transmit system and the RF shield, or adjacent to the conductors of the RF transmit system, as a means of efficiently utilizing the space within the MR bore. By reducing the conductivity or availability of the RF current pathways on the inserted structures, the structures can be placed between the RF conductors of the coil system and the RF shield without degrading the transmit efficiency of the RF transmit system. Presented embodiments focus on hybrid PET/MR systems, and include a method for adapting an RF transmit system to open space between the conductors for sensors and or actuators.

## Description

The present invention relates to magnetic resonance imaging, more specifically to combining additional systems into the MRI scanner such as hybrid MR PET.

Magnetic resonance imaging (MRI) is performed on a MRI scanner. A MRI scanner typically consists of a coil system to generate the static magnetic field B0. Next to that, the gradient coils allow spatial encoding on the B0 field which is required for tomographic imaging. Finally, an RF transmit and receive system is required to generate and pick-up the B1 fields. These fields excite the atomic nuclei. Upon relaxation, the nuclei emit signals of the same frequency that are being picked-up by the system. The frequency of these fields is proportional to the B0 field strength (e.g. 64 MHz at 1.5 T for the hydrogen nucleus).

Over the past decades, MRI systems have become a critical tool for imaging the tissue within a human subject. The integration of the MR system with a positron emission tomography (PET) scanner allows the radioisotope-tagged metabolic markers of the PET scanners to be co-registered and displayed on the high-spatial resolution and inherent tissue-contrast resolved MR images, allowing clinicians and researchers to pinpoint structure and function in disease. In order to detect the mutual annihilation collision of the radio-isotopes, a ring of PET detectors must be placed around the region of interest to detect oppositely directed gamma rays (180-degrees apart) resulting from the annihilation collision. In addition to the benefits of combining the derived images, there is also the benefit of conducting the PET experiment at high field, as the path-length to the annihilation collision is reduced to below 1 mm in magnetic fields on the order of the field strengths used in MR systems (A Simulation Study of a Method to Reduce Positron Annihilation Spread Distributions Using a Strong Magnetic Field in Positron Emission Tomography, H. Iida ; I. Kanno ; S. Miura ; M. Murakami ; K. Takahashi ; K. Uemura, IEEE Transactions on Nuclear Science 33(1):597 - 600 · March 1986).

Due to the inherent benefits of combining PET and MR, considerable work has been conducted, leading to commercialized hybrid PET/MR systems, an alternative to in-line or insert approaches. The incorporation of the PET system into the MR system previously has been accomplished through either degradation of the MR coil apparatus or the PET system. Structures such as copper, attenuate the gamma rays, and therefore decrease the signal strength of the PET experiment. Additionally, the electronics of the PET detector must be shielded from or placed outside the region of the RF fields generated by the MR apparatus. The optimal distance between the transmit system and the imaged region is a balance between transmit efficiency and field homogeneity, with increasing distance degrading efficiency. Likewise, alteration of the optimal RF conductors and distance to the shield degrades performance of the transmit coil. At the cost of PET signal attenuation, WO 1991001503 A1, uses light tubes to convey the photons of the PET experiment to the photodetectors of the PET system to reduce deleterious interaction between the MR and PET systems. In US 7218112 B2, the RF conductors of the MR transmit systems are altered from the optimal configuration in order to create a gap for a ring of photodetectors to be centered along the cylindrical bore. In another approach in which compromises to both the MR and PET systems are introduced, US 7218112 B2, the PET photodetectors are incorporated outside and concentric to the RF transmit system and incorporated into openings in the RF shield.
Herein we present an approach that places conductors within the limited physical space between the gradient RF shield and the RF transmit coil of the MR system, with minimal degradation of transmit efficiency. Such an approach can be adapted for use in a hybrid MR/PET system with minimal deleterious interaction between the systems. The system is also applicable for combining MR with other systems (comprised of sensors and/or actuators and supporting electronics) that benefit from line-of site to the imaging target and/or subject.

### Disclosure of the invention

The here described invention relates to a method for incorporating a sensor and/or actuator system within an MRI-bore of an MRI-system. RF coils or antennas are composed of conductive structures contained within an RF shield. By reducing the conductivity of the RF current pathways on the inserted structures, the structures can be placed between the RF conductors of the coil system and the RF shield without substantially degrading the transmit efficiency of the RF coil system. Such a method allows for the limited space to be utilized without disrupting the functioning of the MR system. Presented embodiments focus on hybrid PET/MR systems utilizing birdcage coils, including a method for adapting the birdcage to incorporate more rungs with more narrow trace widths to open space between the conductors for sensors.

An MR integrated system in which shielded sensors and/or actuators are placed between the RF transmit system and the RF shield, or adjacent to the conductors of the RF transmit system. The placement of the sensors/actuators ensures that no or minimal reduction has to be made to the original bore size of the bore size the MR system. The RF transmit system is designed such that its efficiency is not or minimally affected by the placement of the sensors/actuators. An example of such an MR integrated system is an integrated PETMR system, where the PET detectors are placed between the RF shield and a birdcage coil, ensuring no space constraints on the free bore size of the MR system. The birdcage coil is adapted such that the conductive legs are very thin and positioned between the PET detectors, which allows for maximum efficiency for the PET detectors and the RF transmission system.

The method reduces the degree of compromise or degradation that is the typical cost for integrating multi-modal systems. By limiting the main compromise to a minor (less than 50 %) reduction in transmit efficiency of the MR RF transmit system, the performance degradation can be overcome by simply adding additional transmit power.

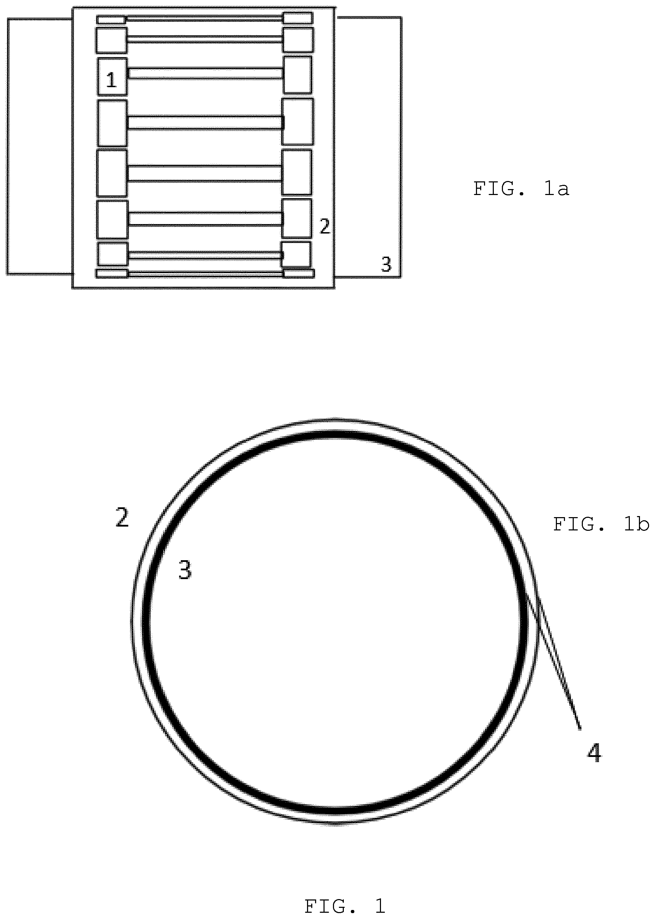

The herein described approach for a hybrid MR system entails utilizing the space between the RF conductors of the transmit system and the RF shield in order to conserve space in such a manner as to not degrade the functioning of the RF transmit system. An embodiment of the critical gap is depicted in Fig. 1, and comprises of the following subcomponents of an MR apparatus: the resonator of an RF transmit system 1, an RF shield (often merged in the gradient coil 2, the bore 3.

Figure 1 details a schematic overview of the relevant subcomponents of the MR system to illustrate the tight space available. The relevant components of the MR system are shown in a side view in Fig 1a, with the system comprising of an example of an RF transmit system 1, the RF shield 2, and the bore of the MR system 3. Figure 1b shows the typical space for a transmit system 1 integrated into the scanner bore 3, and the gap 4 -between the RF shield 2 and scanner bore 3-in which the RF conductors of the transmit system 1 and the sensor and or attenuator system (not depicted) must fit.

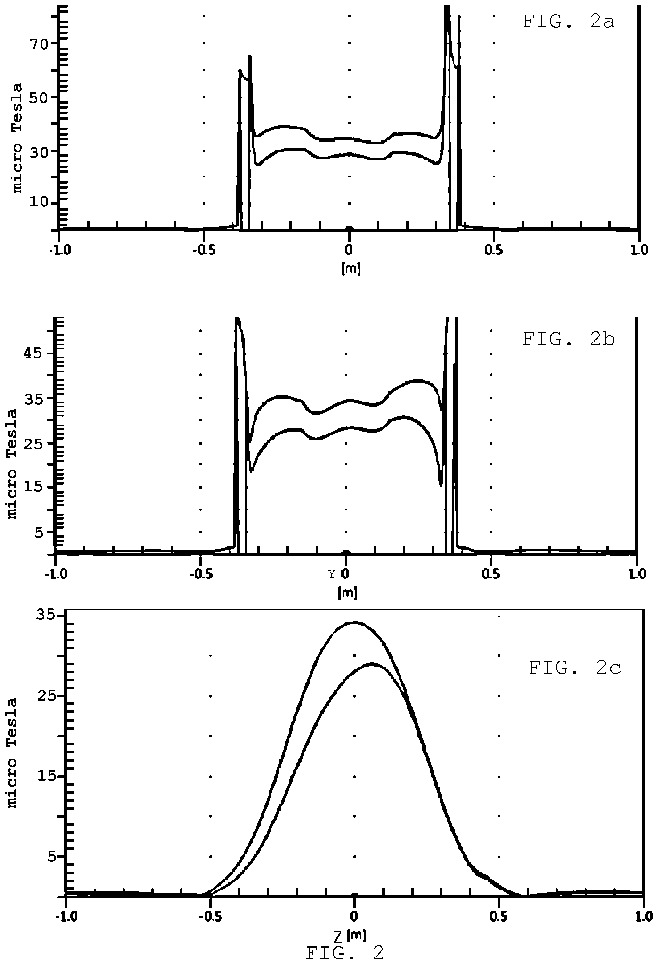

Figure 2 is a presentation of the simulation results comparing the magnitude of the transmit field for the X, Y, and Z directions, respectively 2a, 2b, and 2c.

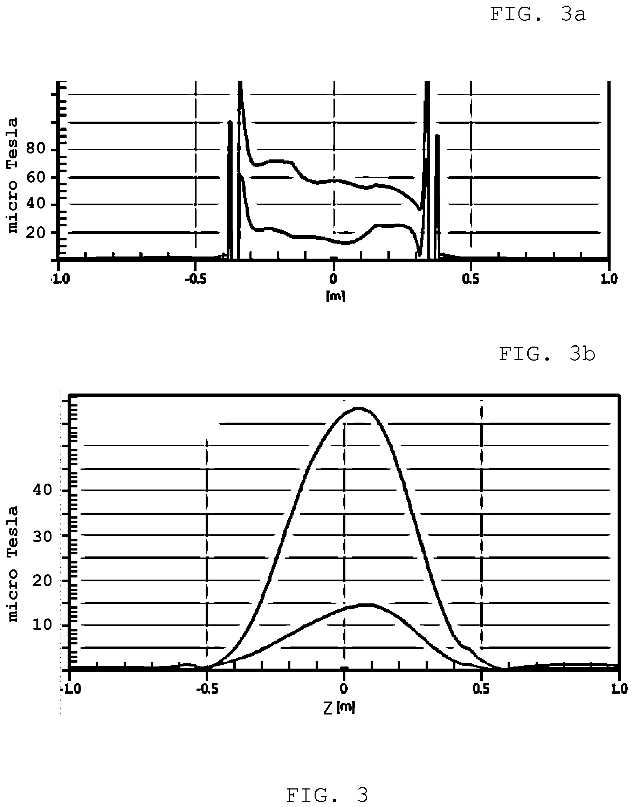

Figure 3 is a presentation of the simulation results for a birdcage resonator with 42-legs and only 21 end ring capacitors showing the magnitude of the transmit field for the X, Y, and Z directions, respectively 3a, 3b, and 3c.

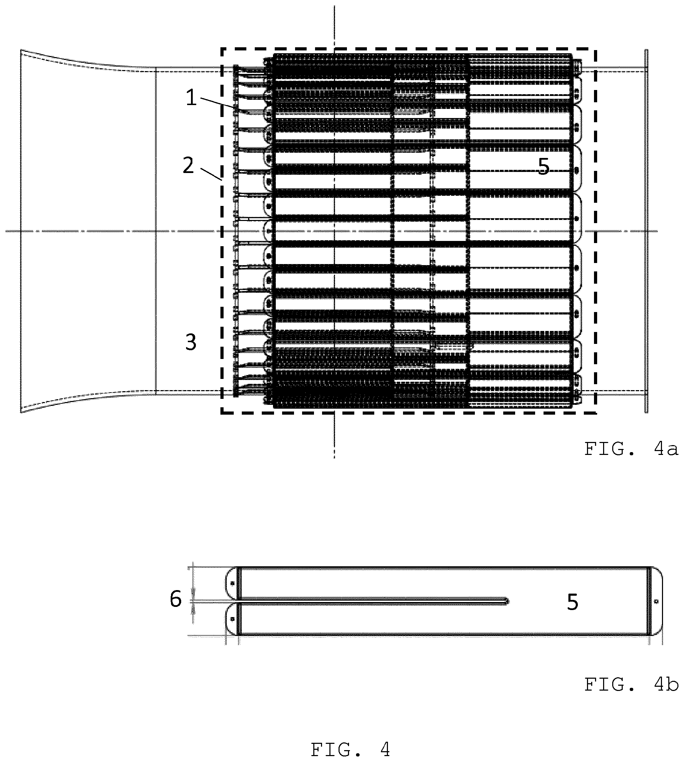

Figure 4 depicts an embodiment of the herein invention showing a modification of a PET detector shielding 5 in which the RF path has been impeded by physically removing the path comprising a slot 6, seen in Fig 4a in an integrated view with a system comprising an RF transmit system 1, RF shield 2, bore 3, and PET shield detectors 5 and in a top view Fig 4b of an example PET detector shield 5.

### Example embodiment for proof of principle:

Simulation studies of transmit efficiency of birdcage coils with and without PET shield detectors, as well as high-pass birdcage simulations with twice as many legs as end ring capacitors. The effect of PET shielding is investigated using Finite Difference Time Domain (FDTD) simulations in Sim4Life (Zurich Med Tech, Zurich), modeling the PET detectors as perfect conductors. In the simulations a high pass birdcage based on a body coil used to model the birdcage. This system operates at 1.5 Tesla (64 MHz). Different geometries of shielded PET detectors are placed around this birdcage to test the effect of the PET shielding on fields produced by the birdcage. These different geometries are compared with a baseline simulation of the birdcage, where no PET detectors are present. The effect of the PET shielding is quantified by extracting the resonance frequency of the homogeneous mode and strength of the B+ 1 field from the simulations. In Fig. 2, the space varying profiles of the |B1+| (y-axis) normalized to 1 w input power on each of two sources from a birdcage coil with and without the modeled PET shield detectors demonstrates that the field profile is only nominally altered, with the delivered |B1+| for a given input power is reduced by less than 20%. However, this represents a worse-case scenario for the modeled geometry because the PET shields are modeled as perfect conductors, whereas in reality, they are fabricated of composite materials with significant resistance. In Fig. 3, function of the RF transmit coil is verified despite alteration to allow more space for the embedded sensor and/or actuator system, where conductive trace widths of the legs are reduced by incorporating additional current paths (simulated as reduced number of capacitors in the end rings).

### Short description of figures:

Fig 1. Depicts an overview of the relevant MR components and illustrates an embodiment of the intended space for integrating a system of sensors and or actuators.
Fig 2. Presents simulation results presenting proof-of-concept that the transmit efficiency of the RF transmit system is degraded by less than 50%.
Fig 3. Presents simulation results demonstrating that a birdcage coil implemented according to Claim 4 still functions.
Fig. 4. Is an embodiment of the herein described invention in which a PET detector is modified for integration in between the RF transmit system conductors and RF shield for PET MR.

## Claims

1. A hybrid MR system comprising an RF shield, a RF transmit system of either coils or antennas that are composed of conductors and shields, and a system of additional sensors and/or actuators and the support electronics, in which the system of additional sensors/actuators are placed between the RF transmit system and the RF shield or adjacent to the RF transmit conductors in such a manner that the RF transmit system is still functional.

2. Placing of shielded devices between conductors and shields of an RF coil or antenna in order to minimize space requirement and constraints.

3. Reducing of conductivity and or availability of RF current pathways in the shields of devices placed between conductors and shields of an RF coil or antenna or adjacent to the conductors in order to maximize the efficiency of the RF coil or antenna within the hybrid MR system.

4. An adapted birdcage resonator for hybrid MR system comprising of an RF transmit system of either coils or antennas that are composed of conductors and shields, and a system of additional sensors and/or actuators and the support electronics, in which the system of additional sensors/actuators are placed between the RF transmit system and the RF shield or adjacent to the conductors in such a manner that the RF transmit system is still functional. Such an adaptation entails replacing wide-trace birdcage legs with a multiplicity of more narrow traces in order to open space between the conductors and allow for accommodating the additional system of sensors and or actuators.

5. A hybrid MR system as described in Claim 1, wherein the system of additional sensors/actuators comprises of a shielded PET detectors wherein the shielded PET detectors are placed between the conductors of the RF transmit system and the shield or adjacent to the conductors of the RF transmit system comprising either coils or antennas for use of PET with MRI.

6. A hybrid PET MR system according to claim 5, wherein the placement of the PET detectors between the shield and conductor of the RF transmit system or adjacent to the RF transmit conductors allows for merged PET with MRI without or with minimum reduction of the free bore size of the non-integrated MRI.
